# EUROPEAN PATENT APPLICATION

(11) **EP 4 847 149 A1**
(43) Date of publication of application: **02.12.2026**
(21) Application number: 25179094.5
(22) Date of filing: 27.05.2025
(51) Int. Cl.: B05C 17/005

(54) **PISTON AND BACK CAP**

(71) Applicant: medmix Switzerland AG, 9469 Haag (Rheintal) (CH)
(72) Inventor: MOSER, Josef, 9469 HAAG (CH)
(74) Representative: Manitz Finsterwald Patent- und Rechtsanwaltspartnerschaft mbB

(57) **Abstract**

The invention relates to a piston configured to be inserted into a cartridge, the piston comprising an outer skirt having at least a first sealing lip at a dispensing end thereof, the piston further comprising a central body forming a dispensing surface of the piston with the dispensing surface having the shape of a truncated cone. The invention further relates to a back cap configured to be connected to a cartridge, in particular to a rear end of the cartridge, with the back cap comprising a sealing section and at least one connection section, wherein the connection section is configured to snap lock the back cap to the cartridge.

## Description

The invention relates to a piston configured to be inserted into a cartridge and to a back cap configured to be connected to a cartridge.

Cartridges that are filled from the front by moving a piston from a front position towards a back position are widely used and commonly known.

However, with these conventional systems often the same problem arises which is air that is trapped inside the cartridge after it has been filled. The presence of air inside the cartridge can lead to a faster deterioration of the material that is stored inside said cartridge.

It is therefore an object of the invention to provide a piston with which the amount of air trapped in a cartridge can be reduced upon filling of material into a cartridge such that no or at least as less as possible air is trapped inside said cartridge when it is stored. Furthermore, it is an object of the invention to provide a back cap with which said filled cartridge can be closed and sealed efficiently once it has been filled brought into a storage state such that no air can enter the cartridge from the back of the cartridge.

This object is solved by the piston and back cap according to the invention.

The piston according to the invention is configured to be inserted into a cartridge. It further comprises an outer skirt having at least a first sealing lip at a dispensing end thereof and a central body forming a dispensing surface of the piston with the dispensing surface having the shape of a truncated cone.

By providing the sealing lip at the dispensing end of the piston, said piston according to the invention is configured in such a way that it minimizes the intake of air upon filling of a cartridge.

Also, the shape of a truncated cone can further provide for a better fitting of the piston into standard cartridges as they often comprise a cone shaped inner surface of the dispensing end thereof in order to be able to press almost all remains of the material stored inside the cartridge towards a dispensing opening. With a piston that is shaped complementary to said inner shape of the cartridge, i.e. that comprises the shape of a truncated cone, said piston can be fitted more accurately into the cartridge to both be able to dispense all of the material stored inside the cartridge and further to avoid trapped air inside the cartridge before and upon filling of the cartridge. This is because the truncated cone can urge the material towards its center and thus towards an outlet.

According to an embodiment of the piston the central body and the outer skirt are two distinct parts that are connectable to one another. This may help ventilating the piston upon filling or dispensing of material as there might be some play between the two components.

According to another embodiment the outer skirt at least partially surrounds the central body. That is, the outer skirt might be arranged around the central body such that the outer skirt, in particular completely, receives the central body.

It may further be possible that the central body is movable relative the outer skirt. Such a movement may comprise both an axial as well as a rotational movement of the central body relative to the outer skirt and may contribute to a better ventilation of the piston.

In this connection it may further be possible that the central body is axially movable relative to the outer skirt. Such a movement can for example arise during the filling or dispensing process of the cartridge when the piston is axially moved to the front or the back of the cartridge.

According to another embodiment a dispensing surface of the truncated cone comprises a radius in the range of 0.2 to 3 mm, in particular a radius in the range of 0.3 o 1.5 mm.

According to a further embodiment the central body further comprises a connection part with a connection surface, wherein the connection surface faces the outer skirt. That is, especially when the central body and the outer skirt are two distinct pieces the central body can comprise a connection part with a connection surface that may be configured to connect the central body to the outer skirt. Nevertheless, this embodiment can also be incorporated when the central body and the outer skirt are made of one single piece.

According to another embodiment the sealing lip completely surrounds the piston such that the piston can seal the entirety of the cartridge's inner surface.

According to a further embodiment the piston may further comprise a second sealing lip at an opposite end of the piston. This may help to further increase the sealing properties of the piston according to the invention such that no air can enter the cartridge at least at the position of the piston.

In this connection it may further be possible that the first and the second sealing lip are facing in opposite directions which may even further increase the sealing properties.

Also by having the sealing lips facing opposite directions an application of a dispensing system or a back cap to the back of the piston can be facilitated.

According to another embodiment the second sealing lip is arranged at the outer skirt in order to provide a sealing contact between the outer skirt and the cartridge.

In this connection it may be possible that the first and second sealing lips are arranged at different ends of the outer skirt. In particular, they might face different directions.

According to another embodiment the outer skirt comprises a first stop surface at an inner surface thereof, facing the dispensing end of the piston, and the central body comprises a second stop surface facing the first stop surface, wherein the first and second stop surfaces are configured to rest on one another at least during a dispensing motion of the piston. Said stop surfaces may be configured to limit an axial motion of the central body relative to outer skirt. Also, they might be configured to align the central relative to the outer skirt.

According to another embodiment the outer skirt further comprises a sealing surface arranged in parallel to a central axis of the piston and facing an outer surface of the central body. The sealing surface might be configured to seal a part between the central body and the outer skirt of the piston such that air that might be trapped in the cartridge at the beginning of a filling process can exit the cartridge through a back end of the cartridge.

It may further also be possible that the sealing surface faces the connection surface of the connection part. Said two surfaces might even contact each other and/or rest against one another.

In this connection it should also be noted that the sealing surface of the outer skirt and the connection surface of the central body may be configured to be connected to one another. Also, the sealing and the connection surface might be configured to ventilate the piston during a filling and/or dispensing process of the cartridge.

According to a further embodiment the sealing surface comprises a wall thickness in the range of 0.1 to 1.5 mm, in particular of 0.2 to 0.6 mm. That is, the wall thickness of the sealing surface might preferably be small enough to be able to ventilate the piston upon a filling and/or dispensing procedure of the cartridge.

In this connection it may further be noted that the sealing surface may comprise at least one wall segment with a reduced wall thickness. Hence, it may be possible for the sealing surface to comprise a varying wall thickness along its circumference by for example comprising at least one wall segment with a reduced wall thickness compared to the regular wall thickness of the sealing surface.

According to a further embodiment said reduced wall thickness of the wall segment lies in the range of 2/10 to 6/10 of the wall thickness of the sealing surface. Such ratios between the regular wall thickness and the reduced wall thickness have proven to provide excellent ventilating properties while simultaneously providing good sealing properties as well.

According to another embodiment the outer skirt comprises two or more wall segments having the reduced wall thickness. In this connection it should be noted that the two or more segments may either all comprise the same (reduced) wall thickness or different thicknesses. The precise thickness of the wall segments may depend on different factors such as the viscosity of the material to be stored, the diameter of the piston and/or the cartridge and so on.

According to a further embodiment the outer skirt, in particular the sealing surface of the outer skirt, comprises at least one connection element configured to movably connect the outer skirt to the central body. The connection element may for example be formed by a projection or the like that is configured to be connected to the outer skirt.

The connection element may further be arranged at an inner surface of the outer skirt, in particular at the sealing surface such that it faces the central body.

According to a further embodiment the central body comprises at least one recess facing the outer skirt. Said recess may either be provided along the entire circumference of the central body or in some cases it might also only be provided at certain segments of the central body.

According to another embodiment the connection element and the recess are configured to act with one another to movably connect the outer skirt to the central body. That is, the connection element may be configured to be inserted into the recess such that it at least axially connects the outer skirt and the central body with one another.

It may also be possible that the recess completely surrounds the central body, in particular the connection surface of the central body.

According to a further embodiment the recess comprises a greater axial height than the connection element such that the central body and the outer skirt can still be axially movable relative to one another even though they are connected with each other.

According to another embodiment the connection element is formed as a projection projecting radially inwardly from the sealing surface, thereby facing an outer surface of the central body, in particular the connection surface of the central body.

In this connection it may be noted that the projection may have a surface inclined with respect to the dispensing end. A projection formed in this way can be used to snap fit the two parts of the piston into one another on assembly thereof and can also be expediently used for venting during a filling process.

According to another embodiment the central body further comprises a nose arranged at the dispensing surface of the central body. Such a nose might for example be necessary in order to close a dispensing nozzle of the cartridge such that no air is trapped inside said nozzle before the cartridge is filled and to further be able to dispense all of the remains of material out of cartridge.

In this connection it is noted that the nose may be configured to seal a dispensing opening of a cartridge.

According to a further embodiment the first and second sealing lips comprise the same degree of flexibility. This helps simplifying the production of the piston as both lips can not only be made out of the same material but also in the same manner.

Alternatively, it can also be possible that the first and second sealing lips comprise a different degree of flexibility which can have an advantageous effect on the sealing properties of the piston.

According to a further aspect of the invention a back cap configured to be connected to a cartridge, in particular to a rear end of the cartridge, is provided. The back cap according to the invention comprises a sealing section and at least one connection section, wherein the connection section is configured to snap lock the back cap to the cartridge. The back cap according to the invention may thus be configured to seal the cartridge, in particular the rear end of the cartridge, in order to be able to store a filled cartridge for a particular period of time without the material stored therein starting to degenerate because of contact with air. Furthermore, the back cap according to the invention can be configured to secure the piston at the rear end of the cartridge to avoid an unintentional removal of the piston at said rear end.

According to an embodiment the sealing section is configured to be at least partially inserted into the cartridge to safely seal the cartridge and/or to secure the piston.

According to another embodiment the sealing section is shaped cylindrically with a flat bottom surface, i.e. it may be pot-shaped which may for example lead to an easier insertion into the cartridge and which may further provide good sealing properties. Also, such a pot-shaped sealing section can be shaped rather complementary to a rear end of the piston, in particular the outer skirt, in order to also be able to (partially) insert the back cap into the piston.

According to a further embodiment the connection section is formed as a wing with said wing being pivotable with respect to the sealing section which allows an easy handling for a user.

It can also be possible that first and second connection sections are provided, with the first and second connection sections both being pivotable with respect to the sealing section.

In this connection it should be noted that the first and second connection sections are both formed as wings.

According to another embodiment the first and second connection sections are arranged at opposite horizontal sides of the back cap such that they can for example be gripped easily by a user.

According to a further embodiment the connection section comprises at least one connection rib extending axially at the connection section, with the connection rib forming a part of a connection recess, with the connection recess being configured to be snapped onto a connection collar of a cartridge. That is, by pivoting the connection section with respect to the sealing section, the connection rib that forms a connection recess which can be put on a connection collar of the cartridge.

In this connection it should be noted that the connection section comprises more than one connection rib forming more than one connection recesses to provide a secure connection between the back cap and the cartridge.

It may also be possible that the connection section comprises a through hole, with said through hole in particular forming part of the connection recess, configured to be snapped onto a connection collar of a cartridge. In this connection it may even be possible that at least parts of the connection collar of the cartridge extend through said through hole when the back cap is connected to the cartridge.

According to a further embodiment the sealing section and the at least one connection section are formed as distinct parts that are connected with one another. Said parts might for example be connected with a joint that allows the pivotable movement of the connection section with respect to the sealing section.

Additionally, a cartridge configured to dispense a material stored therein may be provided with said cartridge comprising a front end and a rear end and a cartridge body extending therebetween, with the front end having a shape of a truncated cone and the rear end comprising a connection collar extending along a circumference of the cartridge body.

According to an embodiment the connection collar comprises a middle part with two flaps extending from the middle part, each flap comprising side faces extending tangentially to said middle part to an end face that connects the two adjacently extending tangential side faces with one another. Such flaps allow for an easier handling for the user since one can hold the cartridge at said flaps between two fingers, for example during a dispensing or filling procedure of the cartridge.

In this connection it should be noted that the middle part may comprise a circular shape that matches an overall shape of the cartridge body.

According to another embodiment the middle part comprises oppositely disposed recesses that are configured to ensure an easy attachment of the cartridge with the back cap according to the invention.

The flaps may further be rounded.

According to another embodiment a radius of a respective end face is larger than a radius of the middle part such that the end face may almost seem straight.

According to a further embodiment the connection collar comprises at least one through hole which may ensure a softer yet safe snap-in of the cartridge with a dispenser.

In this connection it may also be possible that each flap comprises a through hole.

According to another embodiment the cartridge further comprises a nose arranged at the front end of the cartridge and configured to receive a further component such as a cap or a dispensing nozzle. The nose may thus be formed such that it cannot only be used as a dispensing opening but also as a connection part for connecting the cartridge to a further element.

It may for example be possible that the nose comprises an inner tube and an outer tube surrounding the inner tube with the inner tube forming a dispensing outlet and the outer tube forming a connection element.

In this connection the outer tube may for example comprise a thread arranged at an inner side thereof configured to connect the cartridge to a further component such that said further component may simply be screwed onto the cartridge.

According to a further embodiment the cartridge may further comprise a piston, in particular the piston according to the invention, with the piston being configured to urge a material stored inside the cartridge towards the front end and/or to suck a material to be stored through the front end towards the back end of the cartridge.

According to another embodiment the cartridge may further also comprise a back cap, in particular the back cap according to the invention, with the back cap being configured to be attached to the connection collar.

The invention is further described in connection with the exemplary Figures which show:
- Fig. 1A and 1B:: a perspective view of the piston according to the invention;
- Fig. 2: a cross section of the piston according to the invention;
- Fig. 3:: a cross section of the piston arranged in a cartridge;
- Fig. 4:: a further cross section of a piston according to the invention;
- Fig. 5A to 5C:: different views of a back cap according to the invention;
- Fig. 6:: a perspective view of the back cap of Fig. 5;
- Fig. 7:: a cross section of the back cap arranged at a cartridge;
- Fig. 8:: a cross section of a cartridge;
- Fig. 9:: a detailed view of the front end of the cartridge;
- Fig. 10A and 10B:: a back end of the cartridge of Fig. 8;
- Fig. 11:: a detailed view of the back end of Fig. 11; and
- Fig. 12:: a cross section of another piston according to the invention arranged in a cartridge.

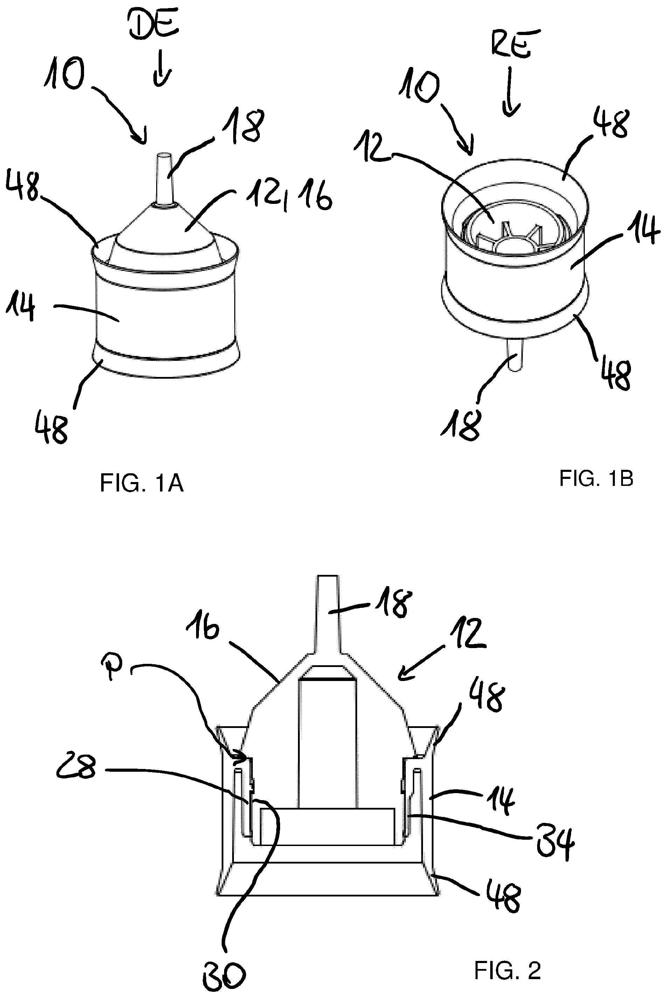

Figures 1A through 4 show different views of a piston 10 according to the invention. Even though the embodiment shown in the Figures shows a piston that is made of two distinct parts, it is nevertheless possible that the piston 10 according to the invention is made out of one single part.

In the Figures it can be seen that the piston 10 comprises a central body 12 as well as an outer skirt 14 that surrounds the central body 12 and that at least partially receives the central body 12.

Both parts comprise a generally circular cross section.

It can further be seen in the perspective view of Fig. 1A that at the central body 12 extends beyond the outer skirt 14 at the dispensing end DE of the piston 10. That is, at least a dispensing surface 16 of the central body 12, which comprises the shape of a truncated cone, extends beyond the outer skirt 14.

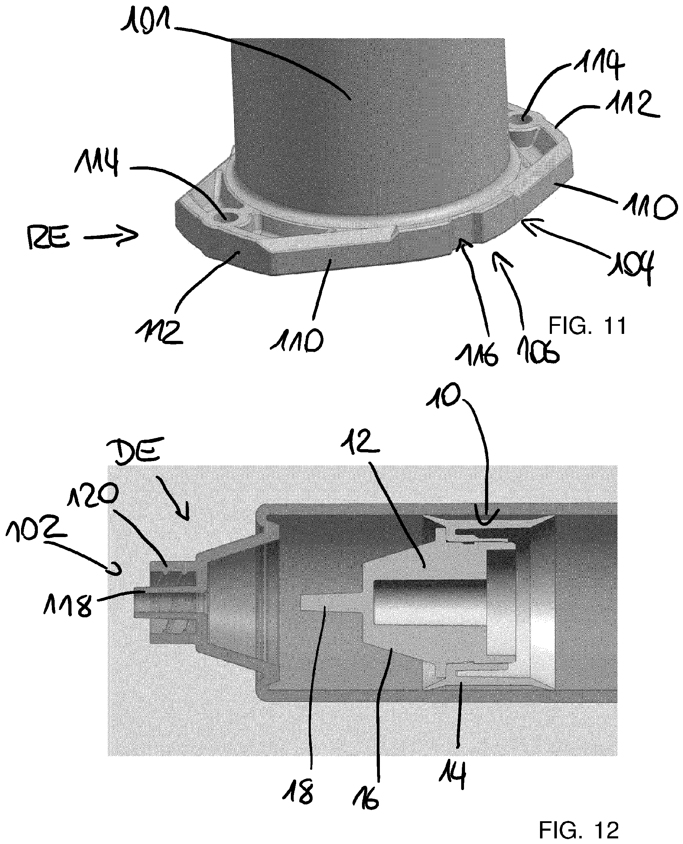

Said dispensing surface 16 can further comprise a nose 18 which is located at the dispensing end DE thereof. Such a nose 18 can for example help minimizing the air intake into the cartridge 100 before filling as it may be inserted into a dispensing nozzle 102 of the cartridge 100 (see e.g. Fig. 12). Furthermore, it can help dispensing all of material stored inside the cartridge 100 as even remains that usually stay inside the nozzle 102 of the cartridge 100 can be dispensed by being urged out of the cartridge 100 by said nose 18.

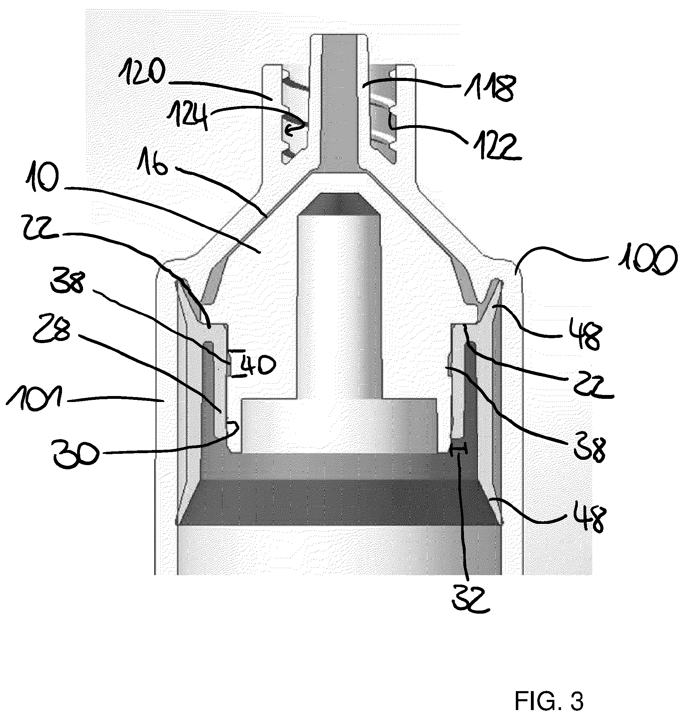

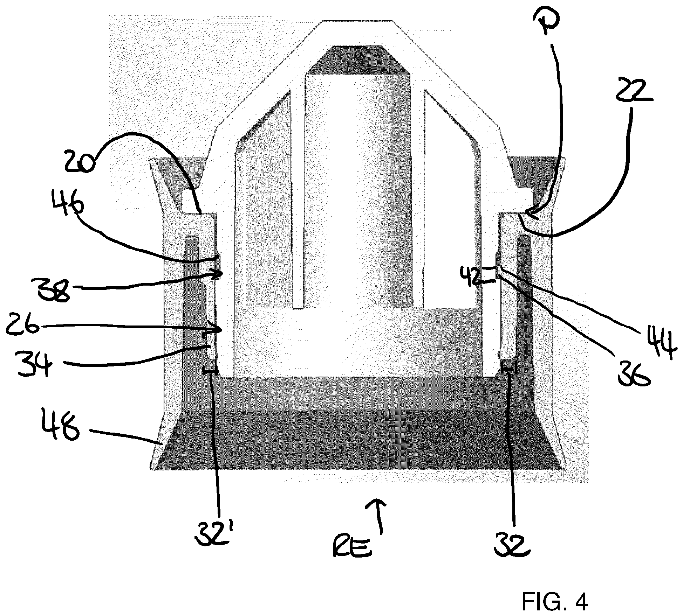

However, the embodiments of Figs. 3 and 4, for example, also show that such a nose 18 is not absolutely necessary and that the piston 10 can also terminate with the dispensing surface 16.

A radius of the dispensing surface 16 at the dispensing end DE thereof can lie in the range of 0.2 to 3 mm. Said radius may in particular lie in the range of a radius of the dispensing nozzle 102 in order to be able to effectively dispense all of the material stored inside the cartridge 100.

Both the outer skirt 14 as well as the central body 12 comprise first respectively second stop surfaces 20, 22 that are configured to rest on one another such that the central body 12 can rest on the outer skirt 14 at least in an axial direction. Both of said stop surfaces 20, 22 can either be formed by a circumferential step which extends along an inner surface 24 of the outer skirt 14 respectively an outer surface 26 of the central body 12.

Alternatively, it could also be possible for the stop surfaces 20, 22 to be formed as two or more distinct elements that are arranged along said surfaces 24, 26.

It can further be seen in the cross sections of Figs. 2 to 4 that the central body 12 and the outer skirt 14 do not only rest on one another on the stop surfaces 20, 22. The outer skirt 14 further comprises a sealing surface 28 which is arranged at the inner surface 24 and which faces the central body 12. Even though the sealing surface 28 is formed integrally with the rest of the outer skirt 14, it can be seen in the Figures that it is formed distinctly from the inner surface 24 of the outer skirt 14.

The central body, on the other hand, comprises a connection surface 30 which is directly located at its outer surface 26.

Said connection surface 30 and sealing surface 28 can be in close contact such that upon movement of the piston 10 in the cartridge 100, no material can flow between said two surfaces 28, 30.

However, the sealing surface 28 can be formed such that upon filling of a cartridge trapped air can flow through said connection part between the two surfaces 28, 30 towards the rear end RE of the piston 10 in order to exit from the interior of the cartridge 100.

This can be accomplished, for example, by choosing an appropriate wall thickness 32 for the sealing surface 28. That is, said wall thickness 32 may be small enough to allow a movement of the sealing surface 28 away from the connection surface 30 when the piston 10 is moved towards the rear end of the cartridge 100 to form a passage way P through which the trapped air can flow.

By forming the passage way P at this position, a particularly beneficial venting of the piston 10 can be achieved. This is because the stop surface 20 of the outer skirt 14 acts as a lowest wall of the passage way P enabling a venting from the lowest point of the sealing lip 48.

However, the wall thickness 32 of the sealing surface 28 also needs to be big enough to ensure that the formed passage way P only allows a flow of air but not of material stored inside the cartridge. That is, the formed passage has to be so narrow that a surface tension of the material stored inside the cartridge keeps the material from entering the formed passage way.

The wall thickness 32 of the sealing surface 28 can lie in the range of 0.1 to 1 mm, in particular in the range of 0.2 to 0.6 mm.

In the embodiment shown in Fig. 4 the above is also achieved by providing a sealing surface 28 which comprises at least one wall segment 34 with a reduced wall thickness 32' compared to the remaining wall thickness 32.

Said one or more wall segments 34 can have a thickness 32' in the range of 2/10 to 6/10 of the wall thickness 32 of the remaining parts of the sealing surface 28.

When more than one wall segment 34 is present, they can be arranged, in particular evenly arranged, along the entire sealing surface 28. Also, it can be possible that all of the wall segments 34 have the same reduced wall thickness 32'.

However, alternatively one could also think of providing a plurality of wall segments 34 with differing reduced wall thicknesses 32' in order to tune the flexibility of the sealing surface 28.

In order to axially connect the sealing surface 28 to the connection surface 30 the sealing surface 28 comprises a connection element 36 that is configured to interact with a recess 38 at the central body 12.

It can be seen that an axial height 40 of the recess 38 is larger than an axial height 42 of the connection element 36 such that upon movement of the piston 10 towards the rear end RE of the cartridge 100 the outer skirt 14 can still axially move away from the central body 12 thereby opening the passage way P by bringing the stop surfaces 20, 22 in a spaced-apart position.

Also, the recess 38 can for example be arranged along the entire circumference of the connection surface 30. Alternatively, one could also think of providing one or more distinct recesses 38 which are spaced apart from one another and arranged along the circumference. In the latter case, the outer skirt 14 can comprise one or more connection elements 36 such that one element 36 interacts with one respective recess 38.

The connection element 36 can further comprise an inclined surface 44 at the dispensing end DE thereof which matches with an inclined surface 46 of the recess 38. This enables a simple assembly of the piston 10 as well as facilitating a filling of the cartridge in which such a piston is stored.

The outer skirt 14 further comprises two sealing lips 16, one at the dispensing end DE and one at the rear end RE of the piston 10, with said sealing lips 16 facing opposite directions.

Said two sealing lips 48 can have the same of differing degrees of flexibility. The exact degree of said sealing lips 48 can be chosen in accordance to the size of the piston for example or the viscosity of the material that is to be stored inside the cartridge 100.

In particular, the degree of flexibility can be chosen such that the cartridge 100 can be effectively sealed by the piston 10 such that no air is trapped inside the cartridge 100.

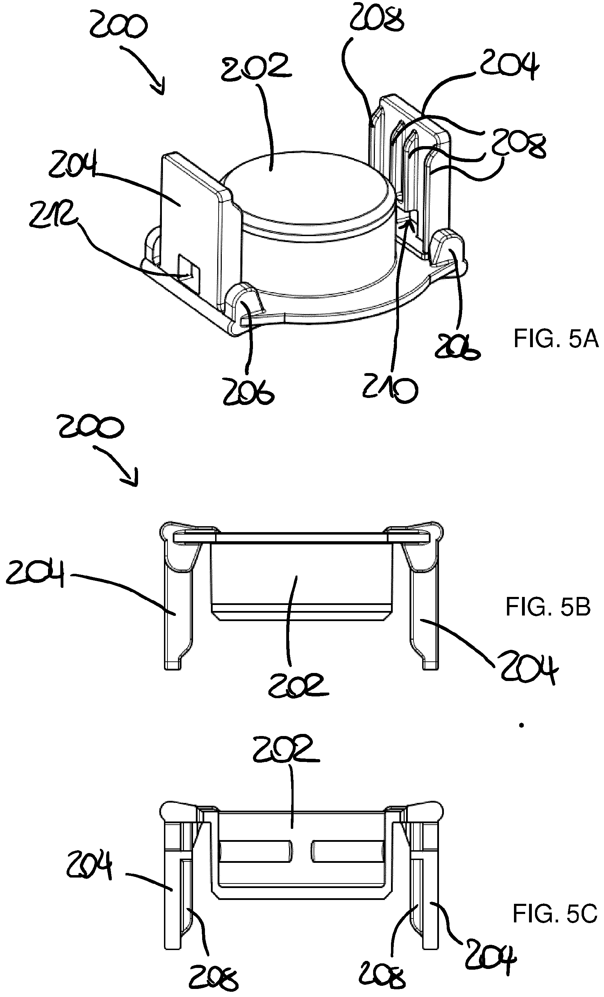

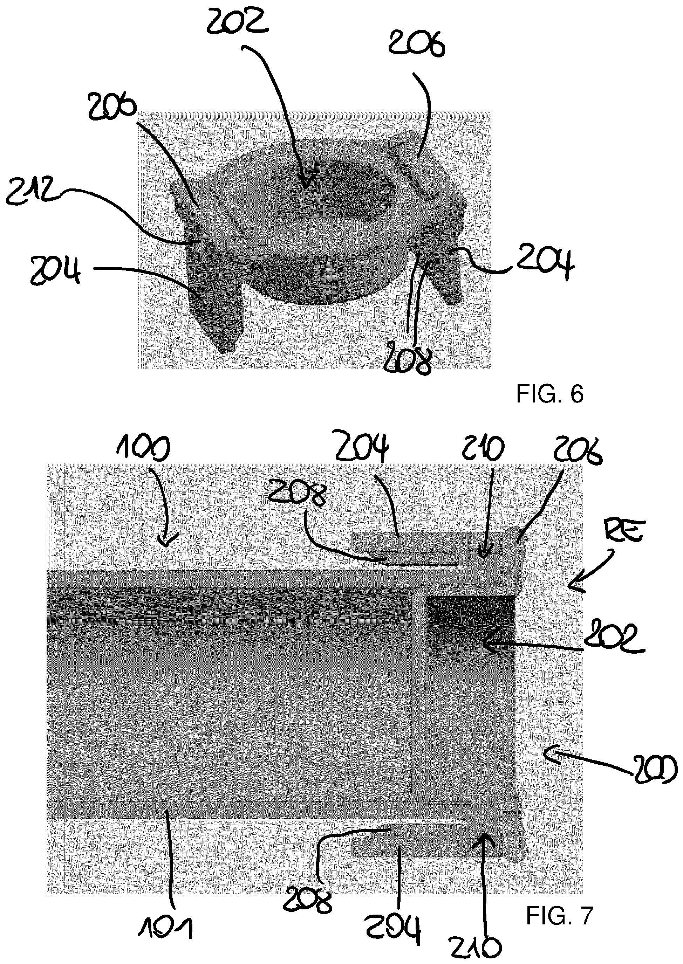

To further ensure a reliable sealing of the cartridge 100, in particular of the rear end RE of the cartridge 100, the invention also provides a back cap 200 having a sealing section 202 and at least one, in the embodiment shown in Figs. 5A to 7 two, connection sections 204, with said connection sections 204 being configured to snap lock the back cap 200 to the cartridge 100 (see Fig. 7).

For this reason, the connection sections 204 are formed as wings that are pivotable with respect to the sealing section 202 by being connected thereto by respective joints 206.

To ensure a rather easy handling, the connection sections 204, i.e. the two wings 204, are arranged at opposite horizontal sides of the back cap 200 as can be seen in the Figures.

The wings 204 are shaped as a square plate with each plate comprising four connection ribs 208, wherein said connection ribs 208 extend axially along a longer side of the square plate and at a surface of the wing 204 that faces the cartridge 100 when the back cap 200 is snap-locked onto the connection collar 104. In the embodiment shown two of the four ribs of each plate 204 form a recess 210 that is configured to be snapped onto a connection collar 104 of the cartridge 100.

The entirety of the connection ribs 208 further provide additional stability to each wing 204 to ensure a higher longevity of the back cap 200 and to further provide a safe connection of the back cap 200 with the cartridge 100.

Each one of the wings 204 further comprise a through hole 212 which forms the connection recess 210. It can even be possible that parts of the connection collar 104 of the cartridge 100 extends through said through hole 212 when the wings 204 are snapped onto the collar 104.

As can also be seen in the Figures, the sealing section 202 of the back cap 200 is generally formed cylindrically with a flat bottom surface, i.e. pot-shaped, such that sealing section 202 can be inserted into the rear end RE of the cartridge 100 and potentially also into the rear end RE of the piston 10, i.e. into the rear sealing lip 16.

Thus, a radius of the pot-shaped sealing section 202 depends on the radius of the cartridge 100 which is supposed to be sealed.

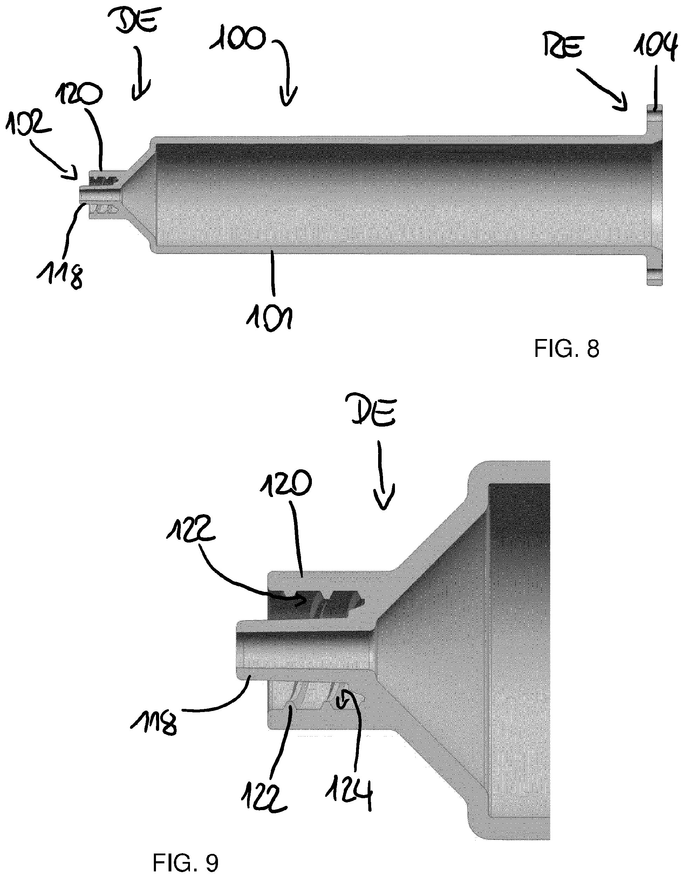

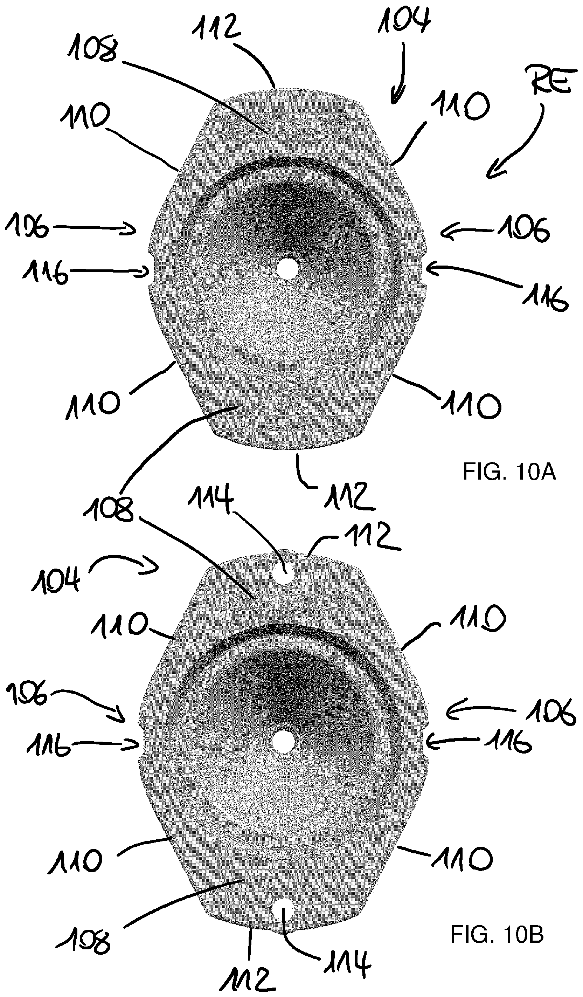

The cartridge 100 which is supposed to be sealed can look like the one shown in Figs. 8 to 11.

The cartridge 100 shown in the Figures is configured to store a material therein and to further receive both the piston 10 as well as the back cap 200 according to the invention. It comprises a front end, i.e. a dispensing end DE, and a rear end RE and a cartridge body 101 extending therebetween.

The dispensing end DE comprises the shape of a truncated cone (just as the piston 10) with a dispensing nozzle 102 being arranged at the front and the rear end RE comprises the above mentioned connection collar 104 which extends along the circumference of the cartridge body 101.

The connection collar 104 comprises a middle part 106 with two flaps 108 extending from said middle part 106.

Said flaps 108 are formed by two side faces 110 that extend tangentially to said middle part 106 which is essentially circular. Furthermore, each flap 108 has an end face 112 which connects the two side faces 110 with one another.

The flaps 108 respectively the end face 112 is rounded. However, a radius of the rounded end face 112 is bigger than a radius of the middle part 106 such that it might almost seem like a straight line.

Each flap 108 can further comprise a through hole 114 which is configured to ensure a safe and soft snap-in of a dispenser.

Also, the middle part 106 comprises two oppositely disposed recesses 116 which are configured to receive the back cap 200, in particular the recess 210 formed by the two middle ribs 208 of the back cap 200. In this connection it is noted that the dimensions of the recesses 116 of the cartridge 100 are chosen such that the back cap 200 can still snap onto the connection collar 104. However, the resistance for the snap-in is lowered such that the handling of the back cap 200 and the cartridge 100 is made easier for the user.

The dispensing end DE of the cartridge 100 comprises - as already mentioned above - the shape of a truncated cone. Furthermore, a nose 116 is arranged at the front which comprises an inner tube 118 and an outer tube 120 with the outer tube 120 surrounding the inner tube 118.

The inner tube 118 is configured to form a dispensing outlet while the outer tube 120 is configured to form a connection element for a potential front cap or a mixer or a dispensing tube or the like.

For this reason, the outer tube 120 comprises a thread 122 arranged at an inner side 124 thereof which is configured to connect the cartridge 100 to said further element by simply allowing said further element to be screwed onto the cartridge 100.

Altogether, it can be seen that with the cartridge 100 and the piston 10 as well as the back cap 200 according to the invention, a system can be provided with which upon front filling of the cartridge 100 the amount of trapped air inside the cartridge 100 can be drastically minimized. This is because of the precise shape and functionality of the piston 10 which provides an effective ventilation when the piston 10 is moved from the front of the cartridge 100 to the back such that potentially trapped air can exit via the rear end of the cartridge 100.

Once the cartridge 100 is completely filled, the back cap 200 according to the invention can be placed on the rear end RE of the cartridge 100 to seal said end and to ensure that no further air can enter the cartridge 100 while it is stored.

### Reference numbers

- 10: piston
- 12: central body
- 14: outer skirt
- 16: dispensing surface
- 18: nose
- 20: stop surface
- 22: stop surface
- 24: inner surface of 14
- 26: outer surface of 12
- 28: sealing surface
- 30: connection surface
- 32: wall thickness
- 32': reduced wall thickness
- 34: wall segment
- 36: connection element
- 38: recess
- 40: axial height of 38
- 42: axial height of 36
- 44: inclined surface of 36
- 46: inclined surface of 38
- 48: sealing lips
- 100: cartridge
- 101: cartridge body
- 102: nozzle
- 104: connection collar
- 106: middle part
- 108: flap
- 110: side face
- 112: end face
- 114: through hole
- 116: recess of 106
- 118: inner tube
- 120: outer tube
- 122: thread
- 124: inner side of 120
- 200: back cap
- 202: sealing section
- 204: connection section/wing
- 206: joint
- 208: connection rib
- 210: recess
- 212: through hole
- DE: dispensing end
- RE: rear end
- P: passage way

## Claims

1. A piston configured to be inserted into a cartridge (100), the piston (10) comprising an outer skirt (14) having at least a first sealing lip (48) at a dispensing end (DE) thereof, the piston (10) further comprising a central body (12) forming a dispensing surface (16) of the piston (10) with the dispensing surface (16) having the shape of a truncated cone.

2. The piston according to claim 1,
wherein the central body (12) and the outer skirt (14) are two distinct parts that are connectable to one another, and/or
wherein the outer skirt (14) at least partially surrounds the central body (12), and/or
wherein the sealing lip (38) completely surrounds the piston (10).

3. The piston according to one of the preceding claims,
wherein the central body (12) is movable relative the outer skirt (14), in particular wherein the central body (12) is axially movable relative to the outer skirt (14).

4. The piston according to one of the preceding claims,
wherein a dispensing surface (16) of the truncated cone comprises a radius in the range of 0.2 to 3 mm, and/or
wherein the central body (12) further comprises a connection part with a connection surface (30), wherein the connection surface (30) faces the outer skirt (14).

5. The piston according to one of the preceding claims,
further comprising a second sealing lip (48) at an opposite end of the piston (10), in particular wherein the first and the second sealing lips (48) are facing opposite directions, and/or
wherein the second sealing lip (48) is arranged at the outer skirt (14), and/or wherein the first and second sealing lips (48) are arranged at different ends of the outer skirt (14).

6. The piston according to one of claims 2 to 5,
wherein the outer skirt (14) comprises a first stop surface (20) at an inner surface (24) thereof, facing the dispensing end (DE) of the piston (10), and the central body (12) comprises a second stop (22) surface facing the first stop surface (20),
wherein the first and second stop surfaces (20, 22) are configured to rest on one another at least during a dispensing motion of the piston (10).

7. The piston according to one of the preceding claims,
wherein the outer skirt (14) further comprises a sealing surface (28) arranged in parallel to a central axis of the piston (10) and facing an outer surface (26) of the central body (12).

8. The piston according to claim 7 and one of claims 4 to 6, wherein the sealing surface (28) faces the connection surface (30) of the connection part, in particular
wherein the sealing surface (28) of the outer skirt (14) and the connection surface (30) of the central body (12) are configured to be connected to one another.

9. The piston according to one of claims 7 or 8,
wherein the sealing surface (28) comprises a wall thickness (32) in the range of 0.1 to 1 mm, in particular to 0.2 to 0.6 mm,
in particular wherein the sealing surface (28) comprises at least one wall segment (34) with a reduced wall thickness (32'), and/or wherein the reduced wall thickness (32') of the wall segment (34) lies in the range of 2/10 to 6/10 of the wall thickness (32) of the sealing surface (28), in particular with the outer skirt (14) comprising two or more wall segments (34) having the reduced wall thickness (32').

10. The piston according to one of claims 2 to 9, in particular to claims 4 to 9, wherein the outer skirt (14), in particular the sealing surface (28) of the outer skirt (14), comprises at least one connection element (36) configured to movably connect the outer skirt (14) to the central body (12), in particular wherein the connection element (36) is arranged at an inner surface (24) of the outer skirt (14), in particular at the sealing surface (28).

11. The piston according to one of claims 2 to 10,
wherein the central body (12) comprises at least one recess (38) facing the outer skirt (14).

12. The piston according to claims 10 to 11,
wherein the connection element (36) and the recess (38) are configured to act with one another to movably connect the outer skirt (14) to the central body (12).

13. The piston according to claim 11 or 12,
wherein the recess (38) completely surrounds the central body (12), and/or wherein the recess (38) comprises a greater axial height (40) than the connection element (36).

14. The piston according to one of claims 10 to 13,
wherein the connection element (36) is formed as a projection projecting radially inwardly from the sealing surface (28), in particular wherein the projection (36) having a surface (42) inclined with respect to the dispensing end (DE).

15. The piston according to one of the preceding claims,
wherein the central body (12) further comprises a nose (18) arranged at the dispensing surface (16) of the central body (12), in particular wherein the nose (18) is configured to seal a dispensing opening of a cartridge (100).

16. The piston according to one of claims 5 to 15,
wherein the first and second sealing lips (48) comprise the same degree of flexibility, or
wherein the first and second sealing lips (48) comprise a different degree of flexibility.

17. A back cap configured to be connected to a cartridge (100), in particular to a rear end (RE) of the cartridge (100), with the back cap (200) comprising a sealing section (202) and at least one connection section (204), wherein the connection section (204) is configured to snap lock the back cap (200) to the cartridge (100), in particular wherein the sealing section (202) is configured to be at least partially inserted into the cartridge (100), and/or wherein the sealing section (202) is shaped cylindrically with a flat bottom surface.

18. The back cap according to claim 17,
wherein the connection section (204) is formed as a wing (204) with said wing (204) being pivotable with respect to the sealing section (202), and/or wherein first and second connection sections (204) are provided, with the first and second connection sections (204) both being pivotable with respect to the sealing section (202), in particular wherein the first and second connection sections (204) are both formed as wings (204), and/or wherein the first and second connection sections (204) are arranged at opposite horizontal sides of the back cap (200).

19. The back cap according to one of claims 17 or 18,
wherein the connection section (204) comprises at least one connection rib (208) extending axially at the connection section (204), with the connection rib (208) forming a part of a connection recess (210), with the connection recess (210) being configured to be snapped onto a connection collar (104) of a cartridge (100), in particular wherein the connection section (204) comprises more than one connection rib (208) forming more than one connection recesses (210), and/or
wherein the sealing section (202) and the at least one connection section (204) are formed as distinct parts that are connected with one another.
